# EUROPEAN PATENT SPECIFICATION

(11) **EP 3 960 220 B1**
(45) Date of publication and mention of the grant of the patent: **05.11.2025**
(21) Application number: 20193819.8
(22) Date of filing: 01.09.2020
(51) Int. Cl.: A61M 5/142, A61M 5/145, A61M 5/24

(54) **FIXATION OF RESERVOIR OF DRUG DELIVERY DEVICE**
FIXIERUNG EINES VORRATSBEHÄLTERS EINER ARZNEIMITTELABGABEVORRICHTUNG
FIXATION DE RÉSERVOIR DE DISPOSITIF D'ADMINISTRATION DE MÉDICAMENTS

(43) Date of publication of application: 02.03.2022
(60) Divisional application: 25200260.5
(73) Proprietor: TecMed AG, 3400 Burgdorf (CH)
(72) Inventor: Bosshard, Simon Martin, 3006 Bern (CH); Hanimann, Michael, 3012 Bern (CH); Margot, Roland, 3076 Worb (CH); Streit, Ursina, 3322 Schönbühl (CH); Baumert, Jan, 3455 Grünen (CH)
(74) Representative: Meier Obertüfer, Jürg

(56) References cited:
- EP-A1- 3 260 149
- EP-A1- 3 539 592
- US-A1- 2007 021 718
- US-B2- 8 118 781

## Description

### FIELD OF THE INVENTION

The present invention relates to a medicament delivery devices for infusing, injecting, delivering, administering or dispensing substances and/or liquids such as insulin or hormone preparations. It departs from a drug delivery device with two housing portions connectable to each other to hold a reservoir with the substance inside the housing.

### BACKGROUND OF THE INVENTION

There are several solution known in the art for holding a reservoir or cartridge inside a drug delivery device, for example, inside an infusion drug pump or inside a cartridge holder of an injection pen.

For a precise setting and dispensing of a dose of a medication it is crucial that the reservoir is reliably hold inside the drug delivery device. Any movement of the reservoir relative to the housing of the drug delivery device has to be prevented. If the reservoir would be shifted, in particular in a dose dispensing direction, the plunger inside the reservoir would not be moved according to the set dose. If, for example, the reservoir is not fixed relative to the housing it may be shifted together with the plunger in the dispensing direction and thus the plunger within the reservoir is not moved according to the desired dosage. In this case, the dose dispensed out of the reservoir does not correspond to the dose set. Such inaccuracy may lead an underdosing or overdosing which can have serious consequences for the user.

To be able to hold the reservoir non-movable inside the delivery device or inside a reservoir holder attached to the delivery device prior art approaches use elastic elements such as mechanical springs or deformable rubber or elastomer elements which are arrange on one or both supporting ends of the reservoir. These elastic elements are supposed to compensate mechanical tolerances such that the reservoir can be fixed inside a housing free of play.

US 9,149,580, for example, discloses a cartridge holder comprising a compression spring positioned at shoulder portion of a cartridge. The compression spring absorbs an initially applied injection force acting on a piston and therefore on the cartridge. The cartridge holder is further equipped with a counter-bearing element adapted to provide a proximal end stop for the cartridge. Therefore, the cartridge is clamped between the counterbearing element and the compression spring.

EP3257533 discloses an infusion pump comprising a reservoir inside a housing of the pump. The retention of the reservoir inside the housing is ensured in that a collar-shaped projection of a neck part of the housing rests on tapered portion of the reservoir thereby pushing the reservoir against an internal flange of the housing.

EP3277345 discloses a cartridge holder comprising on a needle side portion axial ribs, which are plastically deformed against a cartridge neck portion, when inserting the cartridge in the cartridge holder. At a proximal end of the cartridge holder a proximal support structure is connected to the cartridge holder and comprises proximal holding ribs adapted to engage a cartridge rim portion and to support the cartridge against movement in the proximal direction. The proximal ribs are not plastically deformable but are elastically deformable when mounting the cartridge into the cartridge holder.

EP3260149 describes a cartridge holder with an attachement means to fixate the head portion of the cartridge inside the holder and provide a sealed sterile barrier for access to the septum of the cartridge.

### DESCRIPTION OF THE INVENTION

The invention is defined by the subject-matter of claim 1. Further embodiments are defined in the dependent claims. It is an objective of the present invention to allow an easy mounting of a reservoir inside a drug delivery device and to reliably prevent a movement of the reservoir in a dispensing direction during dispensing of the medication.

This objective is achieved by a housing of a drug delivery device or of a disposable module of the drug delivery device that is adapted to accommodate a reservoir for holding a liquid drug according to the independent claims. Preferred embodiments are evident from the dependent patent claims. According to the invention the module comprises a first housing portion comprising a rigid bearing element for supporting a neck or head portion of the reservoir and a second housing portion comprising an elastic contact element for supporting an end of the reservoir which is opposite the neck or head portion. The first and second housing portion are non-releasably or releasably connectable to each other to an assembled state in which the first and second housing portion form a cavity, space or recess for accommodating the reservoir. The bearing element and the elastic contact element each define a limitation of the cavity in a longitudinal direction of the cavity to hold or support the reservoir free of play inside the cavity such that the reservoir cannot move during dose setting and dose dispensing relative to the housing of the drug delivery device. According to the invention the elastic contact element is injection molded onto a surface of a body of the second housing portion (two-component technique). The material of the elastic contact element is different than the material of the body.

The elastic property of the elastic contact element allows to compress the elastic contact element preferably in the longitudinal direction of the cavity. That means the elastic contact element is able to compensate a change in the length in the longitudinal direction between the rigid bearing element of the first housing portion and an inner surface of the second housing portion. Preferably, in the assembled state the distance in the longitudinal direction between the bearing element and the elastic contact element is chosen such that the elastic contact element is deformed to a predefined degree when a reservoir is accommodated in the cavity. In this compressed state the elastic contact element exerts a biasing force or pretensioning force acting in the longitudinal direction to the reservoir and thereby pushing the head or neck portion of the reservoir against the rigid bearing element. The reservoir is thus reliably clamped or non-movably held in the longitudinal direction inside the cavity of the housing of the drug delivery device.

The elastic properties of the elastic contact element is further advantageous for damping shocks or high acceleration values, for example, if the drug delivery devices drops to the floor. Furthermore, the elastic properties are useful for the assembly of the first and second housing portion. The housing portions, for example, may be connected to each other to an assembled state by plastic welding. In this case, the first and second housing portion are pressed together by applying a holding force such that at least one housing portion is elastically deformed in the longitudinal direction. After the welding process the holding force is removed and the deformed housing portion relaxes to its initial form. When the housing portion moves back after the welding process into its initial form the elastic contact element compensates the corresponding change in length of the cavity and is still capable of exerting a pretensioning force that is high enough to reliably hold the reservoir within the housing. That means the elastic contact element ensures that the reservoir is reliably held inside the module not only during the assembly but also during the entire life cycle of the drug delivery device.

The elastic contact element is injection molded onto a surface of a body of the second housing portion. That means the number of parts that have to be handled and assembled can be reduced, since the elastic contact element is non-releasably connected to, or integral with, the second housing portion. This simplifies the handling and assembly of the housing of the drug delivery device.

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances

The term "infusion system" refers to a drug delivery device with a cannula or needle that remains in the skin of the patient for a prolonged period of time, for example several hours. By contrast, the term "injection system" or "injection device" is to be understood as a delivery device that is removed from the injection site after each medication event or drug delivery process.

The infusion system may be, for example, a conventional medical drug pump such as an insulin pump with tubing or it may be a drug patch pump without tubing that is attachable directly onto the skin of the user. The injection system may be, for example, a medication injection pen or a pen-shaped injection device.

The term "longitudinal", with or without "axis", refers to a direction or an axis through the device or components thereof in the direction of the longest extension of the device or the component. The terms "radial" refers to a direction through the device or components thereof in a direction generally perpendicular to the longitudinal direction.

The module comprises a first and a second housing portion that envelop the reservoir in the assembled state. The first and second housing portion may be inside the module or the housing portions may form at least a part or the whole outer cover of the module. However, the term "housing" is not limited to an outermost element of the drug delivery device. Preferably, the first and/or the second housing portion form at least a part of the outer cover of the drug delivery device.

**In** a preferred embodiment the module is a disposable module of the drug delivery device. The drug delivery device may comprise the above mentioned module and a reusable module, a second disposable module and/or additional modules, units or elements. Preferably, the above mentioned module may form at least a part of the drug delivery device.

The bearing element is adapted to support, hold or abut a head or neck portion of the reservoir. For this, the bearing element may be realised in form of a curved or plane contact surface, a contact element or a flat portion inside the first housing portion. The bearing element may be integrally formed with or monolithic build by the first housing portion. Alternatively, the bearing element may be formed as a separate member but not integrally formed with the first housing portion. **In** this case, the bearing element is preferably non-releasably connected or fixed to the first housing portion.

The elastic contact element is adapted to support, hold or abut an end of the reservoir which is opposite the head or neck portion. Thus the elastic contact element supports the rear end of the reservoir, which rear end is furthest away from the outlet in the reservoir for dispensing the medication. Preferably, the elastic contact element is compressed in the longitudinal direction if the first and second housing portion are connected to each other to the assembled state and if a reservoir is present inside the cavity. The compressed elastic contact element is preferably adapted to exert a pretensioning force to reservoir. Therefore, the head or neck portion of the reservoir is pressed against the bearing element and thus the reservoir is reliably non-movable hold inside the cavity of the module.

Preferably, the elastic contact element supports the reservoir only in the longitudinal direction such that the reservoir and in particular the rear end of the reservoir (the end opposite the neck or head portion of the reservoir) is at least in a limited range free to move in a plane perpendicular to the longitudinal direction.

The term "play free" is to be understood that the reservoir cannot move inside the module relative to the first and second housing portion in the longitudinal direction during normal use, e.g. during dose setting and dose dispensing. That does not exclude that the first or second housing portion may be slightly (up to several millimeters) deformed during the assembly process, for example, during connecting the first and second housing portion with each other.

During assembly or manufacturing of the complete module of the drug delivery device the first and second housing portion are connected or attached to each other to the assembled state in which the first and second housing portion form a cavity for accommodating the reservoir. The connection between the first and second housing portion is either releasable (for example by a snap-fit connection) or non-releasable (for example by an adhesive or by welding). After the first and second housing portion are connected to each other a cavity or cavity is formed adapted to accommodate the reservoir inside the module. For example, the cavity may have essentially the form of a cylinder, a rectangle or a polygonal. Preferably, in the assembled state the first and second housing portion completely envelop the reservoir. Thus in the assembled state the reservoir cannot be accessed or removed from the module from outside.

The elastic contact element is injection molded onto a surface of a body of the second housing portion. That means during the production of the second housing portion the elastic contact element is inextricably or non-releasably connected or firmly bonded to the body of the second housing portion but the elastic contact element is made of a material different than the body of the second housing portion. For example, the elastic contact element can be essentially made of silicone, rubber or any thermoplastic elastomer whereas the body of the second housing portion may be essentially made of rigid plastic, for example thermoplastic.

The technique used for the production of the second housing portion leads to a component or a material that is also called twin-component material or two component material (2K material).

However, the second housing portion is not limited to a part comprising only two materials. The second housing portion may also made of three or more different materials. The body of the first and/or second housing portion may also made by injection molding. The elastic contact element may be injection molded, for example, in the same production step as the body of the second housing portion. Alternately, the elastic contact element may be injection molded in a separate production step. In this case the elastic contact element is injection molded onto an already produced body of the second housing portion.

In a preferred embodiment the elastic contact element is compressible in the longitudinal direction of the cavity. This preferably corresponds to the longitudinal direction of the reservoir accommodated in the cavity. The compressible elastic contact element is thus able to exert a pretensioning force against an end of the reservoir accommodated in the cavity such that the head or neck portion of the reservoir is pressed with the pretensioning force against the bearing element. Preferably the elastic contact element is only compressible in the longitudinal direction such that the pretensioning force is exclusively directed in the longitudinal direction.

In an alternative embodiment the elastic contact element can be deformable in other direction, for example, in an inclined direction or in a plane perpendicular the longitudinal direction

Preferably, the elastic contact element is essentially made of or comprises silicone or a thermoplastic elastomer (TPE), for example a rubber material. These materials are very well suitable for injection molding the elastic contact element onto the body, in particular a plastic body, of the second housing portion.

Alternatively, the elastic element can be made of other injection moldable material, for example, thermoset plastic.

In a preferred embodiment the body of the second housing part is essentially made of or comprises plastic material other than thermoplastic elastomer. For example, such materials can be common thermoplastic, thermoset, a non-elastomer thermoplastic, a metal or a composite material.

If the elastic contact element is made of a thermoplastic elastomer and the body is made of metal or a composite material the complete second housing portion is a so-called hybrid material. This can be advantageous because suitable material properties from plastics but also from metal can be used in combination.

Preferably, the second housing portion comprises at least two elastic contact elements which support the rear end of the reservoir which is opposite the neck or head portion of the reservoir. The reservoir can be supported stable with more than one elastic contact elements, in particular with two or more elastic contact elements. In this case the elastic contact elements can be injection molded onto the body of the second housing portion such that the contact elements are circumferentially arranged along a rim portion of the accommodated reservoir.

Alternately, the second housing portion comprises only one elastic contact element, that is, for example, ring-shaped for supporting a rear end of the reservoir.

In a preferred embodiment the elastic contact element has an elongated form which extends in the longitudinal direction of the cavity. The elongated form allows an optimal compression of the contact element.

Preferably, the elastic contact element is cone-shaped. The portion of the cone with the larger diameter contacts the body of the second housing portion and the free end of the cone extends away from the body in the longitudinal direction towards the bearing element. The cone-shaped elastic contact element allows to exactly specify and predetermine the force generated by the compression of the elastic contact element. Namely, the contact element can be designed such that the force generated due to compression is progressive with respect to the degree of compression. That can be advantageous for designing the pretensioning force of the elastic contact element for holding the reservoir within the module.

Preferably, the bearing element is formed by a rigid plastic. Such a rigid plastic is not elastically deformable. Even if almost all material can be minimally deformed before a plastic deformation occurs (Hooke's law) the term "rigid" is to be understood that no essential elastic deformation occurs. An example of a rigid plastic is a common thermoplastic. In contrast to a rigid plastic an elastomer is elastically deformable.

The first housing portion preferably comprises on an inside of the housing portion guiding means or a guiding structure for supporting the reservoir in a radial direction, which is perpendicular to the longitudinal direction. The guiding means are preferably inner walls, radial ribs, cams or protrusions extending inside the housing portion. The guiding means support the reservoir if the first housing portion is deformed in the longitudinal direction, for example during a welding process. Hence, an undesirable tilted position of the reservoir can be reliably prevented by the guiding means.

In a preferred embodiment the first or the second housing portion comprises a blind hole or bore for accommodating the reservoir. The other of the first or second housing portion preferably is formed as a cover or comprises a cover for at least partially closing an inlet of the blind hole. The blind hole and the cover form the cavity for the reservoir. Thus, the reservoir is enveloped by the first and the second housing portion. The cover may comprise an opening for a plunger rod such that the plunger rod runs through the cover to the plunger inside the reservoir. When moving the plunger rod in a dispensing direction the plunger rod moves the plunger to the dispensing end inside the reservoir and thus pushes the substance or medication out of the reservoir.

The assembly with a blind hole and a cover allows an easy assembly of the reservoir and the module.

In the assembled state the first and second housing portion are preferably connected to each other by welding, in particular by plastic welding if the housing portions are essentially made of plastic. Therefore, the first and second housing portion are non-releasably connected or attached to each other. The connecting of the first and second housing portion by welding can easily implemented and allows an economic production of the module.

Alternatively, the first and second housing part can be connected to each other by adhesive, by screws, by rivet joints or by a snap-fit connection.

In a preferred embodiment the drug delivery device is an infusion device, particularly an infusion drug pump. The infusion device preferably comprises a dose and dispensing mechanism with a plunger rod. The plunger rod is adapted to move the plunger within the reservoir for dispensing the medication in the reservoir.

In a preferred embodiment the infusion device is a patch pump comprising a disposable part and a reusable part. In this case, the disposable part may comprise the module according to the invention. The reusable part may comprise the dose and dispensing mechanism.

The invention further relates to the drug delivery device comprising the reservoir. The reservoir may also named as cartridge, carpule or container.

In a further preferred embodiment the reservoir comprises a nut, a cavity or a depression at an end opposite the neck or head portion of the reservoir for at least partially accommodating the elastic contact element in the assembled state.

### BRIEF DESCRIPTION OF THE DRAWINGS

The subject matter of the invention will be explained in more detail in the following text with reference to preferred exemplary embodiments which are illustrated in the attached drawings, in which:
- Fig.1: depicts a perspective view of a patch pump;
- Fig. 2: depicts a sectional side view of the patch pump, wherein the cut runs along the longitudinal axis of the of the patch pump;
- Fig. 3: depicts a sectional side view of reservoir unit of the patch pump;
- Fig. 4: depicts a perspective view of a cover portion of a housing of the reservoir unit;
- Fig. 5: depicts a sectional top view without top cover of the reservoir unit.

The reference symbols used in the drawings, and their primary meanings, are listed in summary form in the list of designations. In principle, identical parts are provided with the same reference symbols in the figures.

### DETAILED DESCRIPTION OF PREFERRED EMBODIMENTS

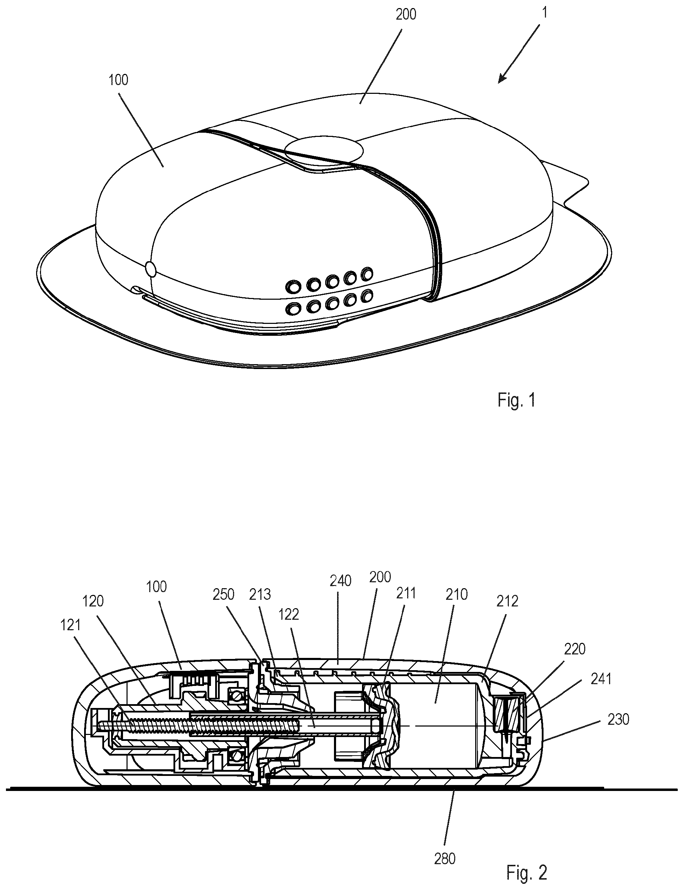

Figure 1 depicts a perspective view of a preferred embodiment of a drug delivery device according to the present invention. The drug delivery device is realised as an infusion patch pump 1. The patch pump 1 comprises a reusable pump unit 100 and a disposable reservoir unit 200. The reservoir unit 200 comprises a reservoir 210 to store a medication and a needle assembly 220 with a fluid path to bring the medication from the reservoir 210 into the body of the patient. At the bottom of the reservoir unit 200 an adhesive patch assembly 280 is included to attach the patch pump 1 to the body of the patient. The pump unit 100 is releasably and sealingly connected to the reservoir unit 200 by a bayonet connection. Figure 1 shows the complete patch pump 1 with both units connected to each other.

As evident from the general description of the embodiment, the user has only two components to assemble: the reusable pump unit 100 with all the components intended for multiple or continuous use, and the disposable reservoir unit 200 with all the components intended for single use.

Figure 2 depicts a sectional view of the patch pump, wherein the cut runs along a middle axis of the patch pump 1. The pump unit 100 comprises a drive mechanism 120 for driving a plunger rod 122, an encoder to supervise the movement of the drive mechanism 120, a rechargeable battery and a programmable electronic system control circuitry configured to control the set-up, drug delivery and supervision of the pump. The battery can be recharged by a further battery in the disposable reservoir unit 200 while the drive mechanism 120 is connected to the reservoir unit 200.

The drive mechanism 120 acts mechanically from a threaded rod 121 via plunger rod 122 on a plunger 211 in the reservoir 210 to dispense the medication out of the reservoir 210. The needle assembly 220 inside the reservoir unit 200 provides the fluid connection from the reservoir 210 to the exterior of the pump for application to the patient.

The reservoir unit 200 comprises a module 230 made of plastic. The module 230 comprises a main portion 240 and a cover portion 250. The main portion 231 comprises a blind hole for accommodating the reservoir 210 and the cover portion 232 covers an opening of the blind hole. In other words, the main portion 240 and the cover portion 250 form a cavity or space for accommodating the reservoir 210 and the reservoir 210 is thus enveloped by the main portion 240 and the cover portion 250 of the module.

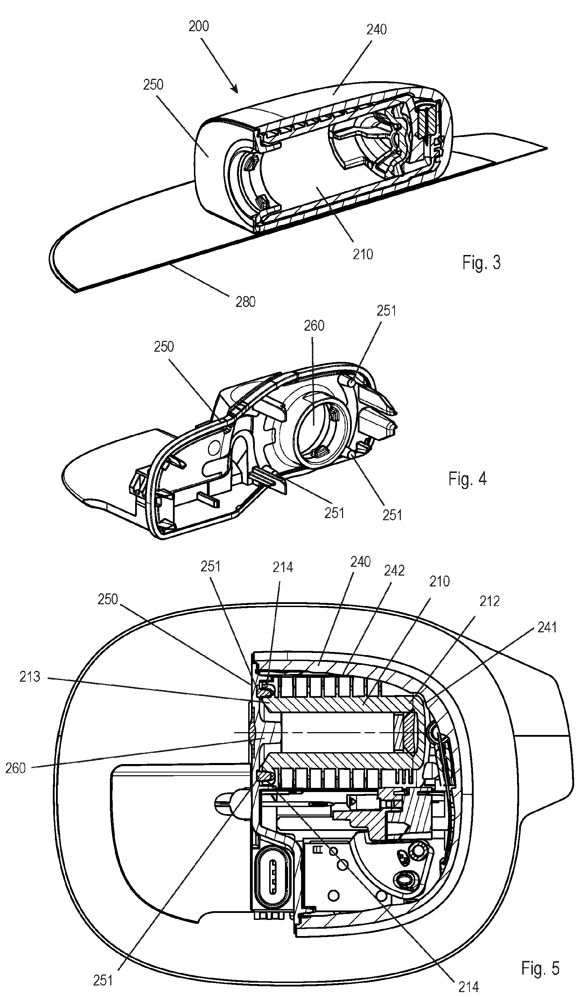

The reservoir 210 has a dispensing end 212 where the needle assembly 220 is arranged. The dispensing end 212 abuts against a rigid bearing surface 241 of the main portion and a rear end 213 opposite the dispensing end 212 abuts elastic contact elements 251 (see figure 4 or 5) of the cover portion 250. In contrast to the elastic contact elements 251 the dispensing end 212 of the reservoir is rigidly supported by the bearing surface 241. which is not elastically deformable. The rear end 213 is supported by the elastically deformable contact elements 251. As described in detail below the reservoir 210 is non-moveably hold or play-free clamped between the bearing surface 241 of the main portion 240 and elastic contact elements 251 of the cover portion 250 (shown in figure 4 and 5).

Figure 3 shows a perspective view of a sectional view of the reservoir unit 200 detached and separated from the pump unit 100. As it can be seen in figure 3 the reservoir unit 200 comprises the adhesive patch assembly 280 at the bottom of the module towards the body of the user. Figure 3 shows the reservoir unit 200 in an assembled state in that the main portion 240 and the cover portion 250 of the module are connected to each other.

Figure 4 depicts a perspective view of the cover portion 250. The cover portion 250 is adapted to be non-releasably connected to the main portion 240 and the body of the cover portion is made of a thermoplastic. The elastic contact elements 251 in form of four cone-shaped protrusions are circumferentially arranged around an opening 260 in the cover portion 250. The contact elements 251 are injection molded onto the body of the cover portion 250 and are made of silicone or thermoplastic elastomer.

In figure 5 a top view of the reservoir unit 200 is depicted as a cross section view (outer housing on top not shown) to allow a view inside the reservoir unit 200. The reservoir unit 200 depicted in figure 5 is sightly enlarged compared to figure 3 and 4. In figure 5, it can be seen that the cone-shaped elastic contact elements 251 protrude in longitudinal direction and are accommodated in corresponding depressions 214 in the rear end 213 or rim portion of the reservoir 210.

During assembly the reservoir 210 is first inserted into the blind hole of the main portion 240 such that the dispensing end 212 of the reservoir 210 abuts the bearing surface 241 of the main portion 240. Subsequently, the cover portion 250 is connected to the main portion 240 portion by plastic welding to cover the blind hole. During this welding process the cover portion 250 is pressed onto the main portion 240 thereby deforming (compressing) the main portion 240 in the longitudinal direction. Upon termination of the welding process the pressure on the cover and main portion 240, 250 is released and the main portions 240 may relax into its initial shape. During this relaxing movement of the main portion 240 the reservoir 210 is radially guided by inner side walls 242 of the main portion 240. The reservoir comprises ribs that may slide along the inner side walls 242 to prevent that the reservoir 210 falls in a blocked or tilted position during deformation of the main portion 240.

Due to the capability of an elastic deformation of the elastic contact elements 251 in the longitudinal direction the reservoir 210 is hold free of play during the welding process and also after relaxation of the housing portions 240, 250 during normal use of the drug delivery device. The dimension of the main portion 240 and the dimension of the elastic contact elements 251 are chosen such that the elastic contact elements 251 are always at least slightly elastically deformed (compressed) even after relaxation of the main portions 240. Therefore, the elastic contact elements 251 compensate the varying length between the supporting points in the longitudinal direction, namely between the elastic contact elements 251 and the bearing surface 241. That means the compressed elastic contact elements 251 always exert a pretensioning force on the rear end 213 of the reservoir 210 and thus press the reservoir 210 against the bearing surface 241. The dispensing end 212 of the reservoir 210 is rigidly supported by the bearing surface 241. That allows to detect an increasing force that acts in the longitudinal direction towards the dispensing end. Such an increasing force can occur, for example, in case of occlusion. Due to the pretensioning force the reservoir 210 cannot move inside the cavity and is hold free of play which allows an reliably and precise dosing and dispensing of the medication.

In a second preferred embodiment the module according to the invention can be implemented in normal infusion pump with tubing. In this second embodiment all features and function described above relating to the module with the main portion 240 and the cover portion 250 comprising the elastic contact elements 251 apply in the same way. The module according to the invention can be implemented without restriction even for an injection system.

While the invention has been described in detail in the drawings and foregoing description, such description is to be considered illustrative or exemplary and not restrictive. Variations to the disclosed embodiments can be understood and effected by those skilled in the art and practising the claimed invention, from a study of the drawings, the disclosure, and the appended claims. In the claims, the word "comprising" does not exclude other elements or steps, and the indefinite article "a" or "an" does not exclude a plurality. The mere fact that certain elements or steps are recited in distinct claims does not indicate that a combination of these elements or steps cannot be used to advantage, specifically, in addition to the actual claim dependency, any further meaningful claim combination shall be considered disclosed.

### LIST OF DESIGNATIONS

- 1: patch pump

- 100: pump unit
- 120: drive mechanism
- 121: threaded rod
- 122: plunger rod

- 200: reservoir unit
- 210: reservoir
- 211: plunger
- 212: dispensing end
- 213: rear end
- 214: depression
- 220: needle assembly
- 230: module
- 240: main portion
- 241: bearing surface
- 242: side wall
- 250: cover portion
- 251: elastic contact elements
- 260: opening
- 280: patch assembly

## Claims

1. Drug delivery device (1) comprising a module adapted to accommodate a reservoir (210) for holding a liquid drug, said module comprising
- a first housing portion (240) comprising a bearing element (241) for supporting a neck or head portion of the reservoir (210)
- a second housing portion (250) comprising an elastic contact element (251) for supporting an end of the reservoir (210) which is opposite the neck or head portion
wherein the first and second housing portion (240, 250) are connectable to each other to an assembled state in which the first and second housing portion form a cavity for the reservoir (210),
wherein the bearing element (241) and the elastic contact element (251) each define a limitation of the cavity in a longitudinal direction of the cavity to hold the reservoir (210) free of play inside the cavity
**characterized in that** the elastic contact element (251) is injection molded onto a surface of a body of the second housing (250) portion and wherein the material of the elastic contact element (251) is different than the material of the body.

2. Drug delivery device (1) according to claim 1, wherein the elastic contact element (251) is compressible in the longitudinal direction of the cavity.

3. Drug delivery device (1) according to any of claims 1 to 2, wherein the elastic contact element (251) comprises a thermoplastic elastomer or silicone.

4. Drug delivery device (1) according to any of claims 1 to 3, wherein the body of the second housing portion (250) is made of plastic material.

5. Drug delivery device (1) according to any of claims 1 to 4, wherein the second housing portion (250) comprises at least two elastic contact elements (251).

6. Drug delivery device (1) according to any of claims 1 to 5, wherein the elastic contact element (251) has an elongated form which extends in the longitudinal direction of the cavity.

7. Drug delivery device (1) according to any of claims 1 to 6, wherein the elastic contact element (251) is cone-shaped.

8. Drug delivery device (1) according to any of claims 1 to 7, wherein the bearing element (241) is formed by a thermoplastic.

9. Drug delivery device (1) according to any of claims 1 to 8, wherein the first housing portion (240) comprises on the inside guiding means (242) for supporting the reservoir (210) in a direction perpendicular to the longitudinal direction.

10. Drug delivery device (1) according to any of claims 1 to 9, wherein one of the first or second housing portion (240, 250) forms a blind hole for accommodating the reservoir (210) and the other of the first or second housing portion (240, 250) forms a cover for at least partially closing an inlet of the blind hole.

11. Drug delivery device (1) according to any of claims 1 to 10, wherein in the assembled state the first and second housing portion (240, 250) are connected to each other by a welding connection.

12. Drug delivery device (1) according to any of claims 1 or 11, wherein the drug delivery device is an infusion device, particularly an infusion drug pump.

13. Drug delivery device (1) according to any of claim 1 to 12, wherein the drug delivery device (1) further comprises the reservoir (210).

14. Drug delivery device (1) according to claim 13, wherein the reservoir (210) comprises a nut or depression (214) at an end opposite the neck or head portion of the reservoir for accommodating the elastic contact element (251) in the assembled state.

## Patentansprüche

1. Arzneimittelabgabevorrichtung (1), umfassend ein Modul, das angepasst ist, um ein Reservoir (210) zum Halten eines flüssigen Arzneimittels aufzunehmen, das Modul umfassend
- einen ersten Gehäuseabschnitt (240), umfassend ein Lagerelement (241) zum Stützen eines Hals- oder Kopfabschnitts des Reservoirs (210)
- einen zweiten Gehäuseabschnitt (250), umfassend ein elastisches Kontaktelement (251) zum Stützen eines Endes des Reservoirs (210), das dem Hals- oder Kopfabschnitt gegenüberliegt
wobei der erste und der zweite Gehäuseabschnitt (240, 250) miteinander zu einem zusammengebauten Zustand verbindbar sind, in dem der erste und der zweite Gehäuseabschnitt einen Hohlraum für das Reservoir (210) ausbilden,
wobei das Lagerelement (241) und das elastische Kontaktelement (251) jeweils eine Begrenzung des Hohlraums in einer Längsrichtung des Hohlraums definieren, um das Reservoir (210) spielfrei im Inneren des Hohlraums zu halten
**dadurch gekennzeichnet, dass** das elastische Kontaktelement (251) auf eine Oberfläche eines Körpers des zweiten Gehäuseabschnitts (250) spritzgegossen ist und wobei sich das Material des elastischen Kontaktelements (251) von dem Material des Körpers unterscheidet.

2. Arzneimittelabgabevorrichtung (1) nach Anspruch 1, wobei das elastische Kontaktelement (251) in der Längsrichtung des Hohlraums komprimierbar ist.

3. Arzneimittelabgabevorrichtung (1) nach einem der Ansprüche 1 bis 2, wobei das elastische Kontaktelement (251) ein thermoplastisches Elastomer oder Silikon umfasst.

4. Arzneimittelabgabevorrichtung (1) nach einem der Ansprüche 1 bis 3, wobei der Körper des zweiten Gehäuseabschnitts (250) aus Kunststoff hergestellt ist.

5. Arzneimittelabgabevorrichtung (1) nach einem der Ansprüche 1 bis 4, wobei der zweite Gehäuseabschnitt (250) mindestens zwei elastische Kontaktelemente (251) umfasst.

6. Arzneimittelabgabevorrichtung (1) nach einem der Ansprüche 1 bis 5, wobei das elastische Kontaktelement (251) eine längliche Form aufweist, die sich in der Längsrichtung des Hohlraums erstreckt.

7. Arzneimittelabgabevorrichtung (1) nach einem der Ansprüche 1 bis 6, wobei das elastische Kontaktelement (251) kegelförmig ist.

8. Arzneimittelabgabevorrichtung (1) nach einem der Ansprüche 1 bis 7, wobei das Lagerelement (241) aus einem Thermoplast ausgebildet ist.

9. Arzneimittelabgabevorrichtung (1) nach einem der Ansprüche 1 bis 8, wobei der erste Gehäuseabschnitt (240) an der Innenseite Führungsmittel (242) zum Stützen des Reservoirs (210) in einer Richtung senkrecht zu der Längsrichtung umfasst.

10. Arzneimittelabgabevorrichtung (1) nach einem der Ansprüche 1 bis 9, wobei einer des ersten oder des zweiten Gehäuseabschnitts (240, 250) ein Sackloch zum Aufnehmen des Reservoirs (210) ausbildet und der andere des ersten oder des zweiten Gehäuseabschnitts (240, 250) eine Abdeckung zum mindestens teilweisen Verschließen eines Einlasses des Sacklochs ausbildet.

11. Arzneimittelabgabevorrichtung (1) nach einem der Ansprüche 1 bis 10, wobei der erste und der zweite Gehäuseabschnitt (240, 250) in dem zusammengebauten Zustand durch eine Schweißverbindung miteinander verbunden sind.

12. Arzneimittelabgabevorrichtung (1) nach einem der Ansprüche 1 oder 11, wobei die Arzneimittelabgabevorrichtung eine Infusionsvorrichtung, insbesondere eine Infusionsarzneimittelpumpe ist.

13. Arzneimittelabgabevorrichtung (1) nach einem der Ansprüche 1 bis 12, wobei die Arzneimittelabgabevorrichtung (1) ferner das Reservoir (210) umfasst.

14. Arzneimittelabgabevorrichtung (1) nach Anspruch 13, wobei das Reservoir (210) an einem Ende gegenüber dem Hals- oder Kopfabschnitt des Reservoirs eine Mutter oder eine Vertiefung (214) zum Aufnehmen des elastischen Kontaktelements (251) in dem zusammengebauten Zustand umfasst.

## Revendications

1. Dispositif d'administration de médicaments (1) comprenant un module adapté pour recevoir un réservoir (210) destiné à contenir un médicament liquide, ledit module comprenant
- une première partie de boîtier (240) comprenant un élément de support (241) pour soutenir une partie du col ou de la tête du réservoir (210)
- une seconde partie de boîtier (250) comprenant un élément de contact élastique (251) pour soutenir une extrémité du réservoir (210) qui est opposée à la partie du col ou de la tête
dans lequel la première et la seconde partie de boîtier (240, 250) peuvent être connectées l'une à l'autre dans un état assemblé dans lequel la première et la seconde partie de boîtier forment une cavité pour le réservoir (210),
dans lequel l'élément de support (241) et l'élément de contact élastique (251) définissent chacun une limite de la cavité dans une direction longitudinale de la cavité pour tenir le réservoir (210) sans jeu à l'intérieur de la cavité
**caractérisé en ce que** l'élément de contact élastique (251) est moulé par injection sur une surface d'un corps de la seconde partie de boîtier (250) et que le matériau de l'élément de contact élastique (251) est différent du matériau du corps.

2. Dispositif d'administration de médicaments (1) selon la revendication 1, dans lequel l'élément de contact élastique (251) est compressible dans la direction longitudinale de la cavité.

3. Dispositif d'administration de médicaments (1) selon l'une quelconque des revendications 1 à 2, dans lequel l'élément de contact élastique (251) comprend un élastomère thermoplastique ou du silicone.

4. Dispositif d'administration de médicaments (1) selon l'une quelconque des revendications 1 à 3, dans lequel le corps de la seconde partie de boîtier (250) est en matière plastique.

5. Dispositif d'administration de médicaments (1) selon l'une quelconque des revendications 1 à 4, dans lequel la seconde partie de boîtier (250) comprend au moins deux éléments de contact élastiques (251).

6. Dispositif d'administration de médicaments (1) selon l'une quelconque des revendications 1 à 5, dans lequel l'élément de contact élastique (251) a une forme allongée qui s'étend dans la direction longitudinale de la cavité.

7. Dispositif d'administration de médicaments (1) selon l'une quelconque des revendications 1 à 6, dans lequel l'élément de contact élastique (251) a une forme conique.

8. Dispositif d'administration de médicaments (1) selon l'une quelconque des revendications 1 à 7, dans lequel l'élément porteur (241) est formé d'un thermoplastique.

9. Dispositif d'administration de médicaments (1) selon l'une quelconque des revendications 1 à 8, dans lequel la première partie de boîtier (240) comprend sur l'intérieur des moyens de guidage (242) pour soutenir le réservoir (210) dans une direction perpendiculaire à la direction longitudinale.

10. Dispositif d'administration de médicaments (1) selon l'une quelconque des revendications 1 à 9, dans lequel l'une parmi la première ou la seconde partie du boîtier (240, 250) forme un trou borgne pour accueillir le réservoir (210) et l'autre de la première ou de la seconde partie de boîtier (240, 250) forme un couvercle pour fermer au moins partiellement une entrée du trou borgne.

11. Dispositif d'administration de médicaments (1) selon l'une quelconque des revendications 1 à 10, dans lequel, à l'état assemblé, la première et la seconde partie de boîtier (240, 250) sont reliées l'une à l'autre par une connexion de soudage.

12. Dispositif d'administration de médicaments (1) selon l'une quelconque des revendications 1 ou 11, dans lequel le dispositif d'administration de médicaments est un dispositif de perfusion, en particulier une pompe à médicaments pour perfusion.

13. Dispositif d'administration de médicaments (1) selon l'une quelconque des revendications 1 à 12, dans lequel le dispositif d'administration de médicaments (1) comprend en outre le réservoir (210).

14. Dispositif d'administration de médicaments (1) selon la revendication 13, dans lequel le réservoir (210) comprend un écrou ou une dépression (214) au niveau d'une extrémité opposée à la partie du col ou de la tête du réservoir pour accueillir l'élément de contact élastique (251) à l'état assemblé.
